# EUROPEAN PATENT SPECIFICATION

(11) **EP 1 427 420 B1**
(45) Date of publication and mention of the grant of the patent: **14.06.2006**
(21) Application number: 02779338.9
(22) Date of filing: 11.09.2002
(51) Int. Cl.: A61K 31/50, A61P 35/00

(54) **USE OF A COMBINATION COMPRISING 4-PYRIDYLMETHYLPHTHALAZINES FOR CANCER TREATMENT**
DIE VERWENDUNG VON EINER 4-PYRIDYLMETHYLPHTALAZINE ENTHALTENDEN KOMBINATION ZUR BEHANDLUNG VON TUMOREN
UTILISATION D'UNE COMBINAISON COMPRENNANT 4-PYRIDYLMETHYLPHTALAZINES POUR LE TRAITEMENT DU CANCER

(30) Priority: 12.09.2001 US 331025 P; 12.09.2001 US 318694 P; 14.09.2001 US 322044 P; 12.06.2002 US 388163 P
(43) Date of publication of application: 16.06.2004
(73) Proprietor: Novartis AG, 4056 Basel (CH); Novartis Pharma GmbH, 1230 Wien (AT)
(72) Inventor: DUGAN, Margaret, Han, Woodside, NY 11377 (US); WOOD, Jeanette, Marjorie, CH-4105 Biel-Benken (CH)
(86) International application number: PCT/EP2002/010194
(87) International publication number: WO 2003/022282

(56) References cited:
- EP-A- 0 722 936
- WO-A-98/35958
- FALBE J.; REGITZ M.: 'Römpp-Lexikon Chemie, Band 2', 1997, GEORG THIEME VERLAG, STUTTGART, NEW YORK * page 865 - page 866 *

## Description

The present invention relates to the use of a 4-pyridylmethyl-phthalazine antiangiogenic agent in combination with chemotherapy by administering agents contemporaneously, separately or sequentially, in particular for use in the treatment of a proliferative disease, especially a solid tumor disease, e.g. renal cancer. The present invention further relates to the use of such a combination for the preparation of a medicament for the treatment of a proliferative disease; to a method of treatment of a warm-blooded animal, especially a human, and improved regimens for the administration of 1-(4-chioroanilino)-4-(4-pyridylmethyl)phthalazine,

4-Pyridylmethyl-phthalazine derivatives that are selective inhibitors of VEGF receptor tyrosine kinase and their preparation, pharmaceutical formulations thereof and methods of making such compounds are described in WO00/59509, EP02/04892, WO01/10859 and, in particular, in U.S. Patent No. 6,258,812, which are here incorporated by reference- Such compounds reduce the microvasculature and inhibit growth of primary tumors and metastases in animal models and are useful for treating diseases associated with deregulated angiogenesis, especially neoplastic diseases (solid tumors), such as breast cancer, cancer of the colon, lung cancer, especially small cell lung cancer, and cancer of the prostate.

Throughout the present invention, the 4-pyridylmethyl-phthalazine derivative is in particular 1-(4-chloroanilino)-4-pyridylmethyl)phthalazine or a pharmaceutically acceptable salt thereof. Studies in humans have shown 1-(4-chloroanilino)+(4-pyridylmethyl)phthalazine to be well tolerated and to reduce tumor vascular permeability. It is understood that further references to 1-(4-chloroanilino)+(4-pyridylmethy)phthalazine are intended to include pharmaceutically acceptable salts thereof.

Chemotherapy for the treatment of proliferative diseases is known in the art.

Surprisingly, it has been found that the antineoplastic effect, in particular in the treatment of a proliferative disease, especially a solid tumor disease, e.g. renal cancer and, in particular, metastatic renal cancer, which is refractory to other chemotherapeutics known as antineoplastic agents, of a combination as defined herein is greater than the effect of a therapy using chemotherapy or a 4-pyridylmethyl-phthalazine derivative alone.

In a preferred embodiment of the present invention, the chemotherapy comprises a platinum compound and an antineoplastic antimetabolite and, optionally, folinic acid. In a specific embodiment of the present invention, the chemotherapy comprises a platinum compound, 5-fluorouracil and folinic acid. In a further specific embodiment of the present invention, the chemotherapy comprises a platinum compound, capecitabine and folinic acid.

The term "antineoplastic antimetabolite" includes, 5-fluorouracil, tegafur, capecitabine, cladribine, cytarabine, fludarabine phosphate, fluorcurldine, gemcitabine, 6-mercaptopurine, hydroxyurea, methotrexate, edatrexate and salts of such compounds, and furthermore ZD 1694 (RALTITREXED^{™}), LY231514 (ALIMTA^{™}), LY264618 (LOMOTREXOL^{™}) and OGT719.

5-Fluorouracil can be prepared, e.g., as described in US 2,802,005. It can be employed in the present invention as marketed, e.g., under the trademark EFUDEX^{™}, FLURACIL^{™} or FLUROBLASTIN^{™}. Tegafur can be employed especially in the form of a composition as disclosed in US 5,116,600 and US 5,525,603. Furthermore, tegafur can be administered, e.g., in the form as it is marketed under the trademarks FTORAFUR^{™}, LAMAR^{™} or NEBEREK^{™}. Capecitabine can be administered, e.g., in the form as disclosed in US 5,472,949 or in the form as it is marketed, e.g., under the trademark XELODA^{™}. Cladribine can be prepared, e.g., as disclosed in US 4,760,135. It can be administered, e.g., in the form as it is marketed under the trademarks LEUSTATIN^{™} or LEUSTAT^{™}. Cytarabine can, e.g., be prepared as disclosed in US 3,116,282 or by Hessler in J. Org. Chem. 41 (1970) 1828. It can be administered, e.g., in the form as it is marketed under the trademarks ARA-C^{™}, CYTOSAR^{™} or UDICIL^{™}. A suitable salt of such compound is cytarabine ocfosfate (STARASID^{™}) which can be prepared as described in US 4,812,560. Fludarabine phosphate can be prepared as described in US 4,357,324. It can be applied as marketed under the trademark FLUDARA^{™}. Gemcitabine can be administered, e.g., in accordance with the disclosure of US 5,464,826 or in the form as it is marketed, e.g., under the trademark GEMZAR^{™}. 6-Mercaptopurine (6-purinethiol) can, e.g., be prepared as disclosed in US 2,933,498. It can be employed as marketed, e.g., under the trademark LEUKERIN^{™} or PURINETHOL^{™}. Hydroxyurea can, e.g., be prepared as disclosed in US 2,705,727. Methotrexate can be employed as marketed, e.g., under the trademark FOLEX^{™} or MTX^{™}. Edatrexate can, e.g., be prepared as disclosed in US 4,369,319.

The term "folinic acid" relates to "N-[4-[[(2-amino-5-formyl-1,4,5,6,7,8-hexahydro-4-oxo-6-pteridinyl)methyl]amino]benzoyl-L-glutamic acid, which is marketed, e.g., under the trademark LEUCOVORIN^{™}.

The term "platinum compound" as used herein means carboplatin, cisplatin or oxaliplatin. Preferably, the platinum compound is oxaliplatin.

The term "carboplatin" as used herein relates to the antineoplastic agent *cis*-diamine (1,1-cyclobutane dicarboxylato) platinum(II), which is disclosed, e.g., in US 4,140,707 or by R.C.

Harrison et al. in Inorg. Chim. Acta 46, L15 (1980). This drug can be administered e.g., in the form as it is marketed, e.g. under the trademark CARBOPLAT^{™} or PARAPLATIN^{™}.

The term "oxaliplatin" as used herein relates to the antineoplastic agent also known as oxalatoplatinum, which is disclosed, e.g., in US 5,716,988. This drug can be administered e.g., in the form as described in the cited US patent or in the form it is marketed, e.g. under the trademark ELOXANTINE^{™} or 1-OHP^{™}.

The term "cisplatin" as used herein relates to the antineoplastic agent also known as *cis-*diaminedichloroplatinum, which compound and its use as antineoplastic agent is disclosed, e.g., in DE 2,318,020.

The term "topoisomerase I inhibitors" as used herein includes, but is not limited to topotecan, irinotecan, 9-nitrocamptothecin and the macromolecular camptothecin conjugate PNU-166148 (compound A1 in WO99/17804). Irinotecan can be administered, e.g., in the form as it is marketed, e.g. under the trademark CAMPTOSAR^{™}. Topotecan can be administered, e.g., in the form as it is marketed, e.g. under the trademark HYCAMTIN^{™}.

In a broader sense of the invention, the term "chemotherapy" refers to the administration of an antineoplastic agent selected from the group that includes, aromatase inhibitors, antiestrogens, topoisomerase II inhibitors, microtubule active agents, protein kinase C inhibitors, gonadorelin agonists, anti-androgens, bisphosphonates, histone deacetylase inhibitors, S-adenosylmethionine decarboxylase inhibitors, and trastuzumab.

In one preferred embodiment of the invention, the antineoplastic agent is selected from the group consisting of aromatase inhibitors, antiestrogens, topoisomerase II inhibitors, microtubule active agents, in particular discodermolide, protein kinase C inhibitors, in particular staurosporine derivatives, gonadorelin agonists, anti-androgens, bisphosphonates, in particular pamidronic acid or zoledronic acid, and trastuzumab. A further preferred embodiment of the present invention pertains to the combination of 4-pyridylmethyl-phthalazine antiangiogenic agent, in particular 1-(4-chloroanilino)-4-(4-pyridylmethyl)-phthalazine, and discodermolide.

The term "aromatase inhibitors" as used herein relates to compounds which inhibit the estrogen production, i.e. the conversion of the substrates androstenedione and testosterone to estrone and estradiol, respectively. The term includes, steroids, especially exemestane and formestane and, in particular, non-steroids, especially aminoglutethimide, vorozole, fadrozole, anastrozole and, very especially, letrozole. Exemestane can be administered, e.g., in the form as it is marketed, e.g. under the trademark AROMASIN^{™}. Formestane can be administered, e.g., in the form as it is marketed, e.g. under the trademark LENTARON^{™}. Fadrozole can be administered, e.g., in the form as it is marketed, e.g. under the trademark AFEMA^{™}. Anastrozole can be administered, e.g., in the form as it is marketed, e.g. under the trademark ARIMIDEX^{™}. Letrozole can be administered, e.g., in the form as it is marketed, e.g. under the trademark FEMARA^{™} or FEMAR^{™}. Aminoglutethimide can be administered, e.g., in the form as it is marketed, e.g. under the trademark ORIMETEN^{™}.
A combination of the invention comprising an antineoplastic agent which is an aromatase inhibitor is particularly useful for the treatment of hormone receptor positive breast tumors.

The term "antiestrogens" as used herein relates to compounds which antagonize the effect of estrogens at the estrogen receptor level. The term includes, tamoxifen, fulvestrant, raloxifene and raloxifene hydrochloride. Tamoxifen can be administered, e.g., in the form as it is marketed, e.g. under the trademark NOLVADEX^{™}. Raloxifene hydrochloride can be administered, e.g., in the form as it is marketed, e.g. under the trademark EVISTA^{™}. Fulvestrant can be formulated as disclosed in US 4,659,516 or it can be administered, e.g., in the form as it is marketed, e.g. under the trademark FASLODEX^{™}.

The term "topoisomerase II inhibitors" as used herein includes, the antracyclines doxorubicin (including liposomal formulation, e.g. CAELYX^{™}), epirubicin, idarubicin and nemorubicin, the anthraquinones mitoxantrone and losoxantrone, and the podophillotoxines etoposide and teniposide. Etoposide can be administered, e.g., in the form as it is marketed, e.g. under the trademark ETOPOPHOS^{™}. Teniposide can be administered, e.g., in the form as it is marketed, e.g. under the trademark VM 26-BRISTOL^{™}. Doxorubicin can be administered, e.g., in the form as it is marketed, e.g. under the trademark ADRIBLASTIN^{™}. Epirubicin can be administered, e.g., in the form as it is marketed, e.g. under the trademark FARMORUBICIN^{™}. Idarubicin can be administered, e.g., in the form as it is marketed, e.g. under the trademark ZAVEDOS^{™}. Mitoxantrone can be administered, e.g., in the form as it is marketed, e.g. under the trademark NOVANTRON^{™}.

The term "microtubule active agents" relates to microtubule stabilizing and microtubule destabilizing agents selected from the group consisting of paclitaxel, docetaxel, eleutherobin, the vinca alkaloids, e.g., vinblastine, especially vinblastine sulfate, vincristine especially vincristine sulfate, and vinorelbine and discodermolide. Vinblastine sulfate can be administered, e.g., in the form as it is marketed, e.g. under the trademark VINBLASTIN R.P.^{™}. Vincristine sulfate can be administered, e.g., in the form as it is marketed, e.g. under the trademark FARMISTIN^{™}. Discodermolide can be obtained, e.g., as disclosed in U.S. patent nos. 4,939,168 and 5,618,487 to Harbor Branch Oceanographic Institute or by chemical synthesis as described, for example, in GB 2280677, WO 98/24429 and U.S. patent nos. 5,789605 and 6,031,133.

The term "protein kinase C inhibitors", refers in particular to staurosporine derivatives, and preferably to those disclosed in US 5,093,330. Such compounds can be administered in the form as disclosed in WO99/48896.

The term "anti-angiogenic compounds" as used herein relates to thalidomide (THALOMID^{™}) and SU5416.

The term "gonadorelin agonist" as used herein includes, abarelix, goserelin and goserelin acetate. Goserelin is disclosed in US 4,100,274 and can be administered, e.g., in the form as it is marketed, e.g. under the trademark ZOLADEX^{™}. Abarelix can be formulated, eg. as disclosed in US 5,843,901.

The term "anti-androgens" as used herein includes, bicalutamide (CASODEX^{™}), which can be formulated, e.g. as disclosed in US 4,636,505.

The term "bisphosphonates" as used herein includes, etridonic acid, clodronic acid, tiludronic acid, pamidronic acid, alendronic acid, ibandronic acid, risedronic acid and zoledronic acid. "Etridonic acid" can be administered, e.g., in the form as it is marketed, e.g. under the trademark DIDRONEL^{™}. "Clodronic acid" can be administered, e.g., in the form as it is marketed, e.g. under the trademark BONEFOS^{™}. "Tiludronic acid" can be administered, e.g., in the form as it is marketed, e.g. under the trademark SKELID^{™}. "Pamidronic acid" can be administered, e.g., in the form as it is marketed, e.g. under the trademark AREDIA^{™}. "Alendronic acid" can be administered, e.g., in the form as it is marketed, e.g. under the trademark FOSAMAX^{™}. "Ibandronic acid" can be administered, e.g., in the form as it is marketed, e.g. under the trademark BONDRANAT^{™}. "Risedronic acid" can be administered, e.g., in the form as it is marketed, e.g. under the trademark ACTONEL^{™}. "Zoledronic acid" can be administered, e.g., in the form as it is marketed, e.g. under the trademark ZOMETA^{™}.

The term "histone deacetylase inhibitors" as used herein includes, MS-275, SAHA, FK228 (formerly FR901228), Trichostatin A and the compounds disclosed in WO 02/22577, in particular NVP-LAQ824 or its lactate salt.

The term "S-adenosylmethionine decarboxylase inhibitors" as used herein includes, the compounds disclosed in US 5,461,076.

"Trastuzumab" can be administered, e.g., in the form as it is marketed, e.g. under the trademark HERCEPTIN^{™}.

The structure of the active agents identified by code nos., generic or trade names may be taken from the actual edition of the standard compendium ''The Merck Index" or from databases, e.g. Patents International (e.g. IMS World Publications).

The present invention further relates to a "combined preparation" comprising (a) a 4-pyridylmethyl-phthalazine antiangiogenic agent, in particular 1-(4-chloroanilino)-4-(4-pyridylmethyl)phthalazine, and (b) one or more chemotherapy agents, in particular oxaliplatin, folinic acid and 5-fluoruracil. The term "a combined preparation", as used herein defines especially a "kit of parts" in the sense that the combination partners (a) and (b) as defined above can be dosed independently or by use of different fixed combinations with distinguished amounts of the combination partners (a) and (b), i.e., simultaneously or at different time points. The parts of the kit of parts can then, e.g., be administered simultaneously or chronologically staggered, that is at different time points and with equal or different time intervals for any part of the kit of parts.

The present invention also relates to the use of a COMBINATION OF THE INVENTION for the preparation of a medicament for the treatment of a proliferative disease.

The inventive combination therapy is especially useful for the treatment of solid tumor diseases. The term "solid tumor diseases" especially means renal cancer, breast cancer, ovarian cancer, cancer of the colon, for example advances colorectal cancer, and generally the Gl tract like, e.g., gastric cancer, cervix cancer, lung cancer, in particular small-cell lung cancer, and non-small-cell lung cancer, head and neck cancer, bladder cancer, cancer of the prostate, Kaposi's sarcoma, carcinoid tumors and carcinoid syndrome. The present combination inhibits the growth of solid tumors and liquid tumors and is also suited to prevent the the metastatic growth of these tumors.

The term "carcinoid tumor" as used herein relates to a neuroendocrine tumor arising from the enterochromaffin cells which cells are scattered mainly throughout the intestine and main bronchi. Peptides synthesized by carcinoid tumors include 5-hydroxytryptamine and 5-hydroxytrypthophan.

The term "carcinoid syndrome" as used herein relates to a disease, in particluar the manifestation of an advanced disease, the symptoms of which include cutaneous flushing, diarrhea and palpable abdominal mass or hepatomegaly. In said disease the urinary concentration of 5-hydroxyindolacetic acid (5-HIAA) typically relates directly to the tumor volume and correlates with the chance of survival. A level of > 8 mg/24 hours of 5-HIAA is a sensitive measurement in 75 % of all cases of carcinoid syndrome. Another indicator for the syndrome is an increased plasma serotonin level, in particluar a plamsa serotonin level higher than about 250, especially 350 ng/ml.

The term "metastatic growth" as used herein comprises the metastatic spread of tumors and the growth and development of micrometastases in other organs of the cancer patients.

It will be understood that references to the combination partners (a) and (b) are meant to also include the pharmaceutically acceptable salts of the compounds.

A combination which comprises (a) at least one antineoplastic agent known in chemotherapy and (b) a 4-pyridylmethyl-phthalazine derivative, in which the active ingredients are present in each case in free form or in the form of a pharmaceutically acceptable salt and optionally at least one pharmaceutically acceptable carrier, will be referred to hereinafter as a COMBINATION OF THE INVENTION.

The nature of proliferative diseases is multifactorial. Under certain circumstances, drugs with different mechanisms of action may be combined. However, just considering any combination of drugs having different mode of action does not necessarily lead to combinations with advantageous effects.

All the more surprising is the experimental finding that *in vivo* the administration of a COMBINATION OF THE INVENTION, results not only in a beneficial effect, especially a synergistic therapeutic effect, e.g. with regard to slowing down, arresting or reversing the neoplasm formation or a longer duration of tumor response, but also in further surprising beneficial effects, e.g. less side-effects, an improved quality of life and a decreased mortality and morbidity, compared to a monotherapy applying only one of the pharmaceutically active ingredients used in the COMBINATION OF THE INVENTION.

The effective dosage of each of the combination partners employed in the COMBINATION OF THE INVENTION may vary depending on the particular compound or pharmaceutical composition employed, the mode of administration, the condition being treated, the severity of the condition being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites.

For the determination of the active dosage of a 4-pyridylmethyl-phthalazine antiangiogenic agent to be applied to patients and, in particular, to be applied to an individual patient, in monotherapy or combination therapy, two biomarkers, DCE-MRI and plasma VEGF level, along with exposure, safety, and tumor response data can be employed. For this purpose, patients, e.g., receive a continuous daily dose of, e.g., 50, 150, 300, 500, 750, 1000, 1500 or 2000 mg of 1-(4-chloroanilino)-4-(4-pyridylmethyl)phthalazine. Pharmacokinetic (PK) samples are taken at predose, and days 1, 15, and 28. DCE-MRI, which reflects the change in tumor perfusion and vascularity, are performed at baseline, day 2, and day 28. For all evaluable MRI scans, contrast enhancement for the whole tumour can be assessed by calculating the bi-directional transfer constant (Ki) and expressed as a percentage of baseline Ki for evaluation on day 2 and 28. Plasma VEGF, a proangiogenic factor produced by the tumor cells, reflects the hypoxic status of the tumor and are sampled at baseline, and on days 1, 8, 15, 22, and 28. A significant relationship exists between the percentage of baseline Ki and an increase in the dose of the 4-pyridylmethyl-phthalazine antiangiogenic agent, its exposure, and its plasma concentration as determined by the Spearman Rank Correlation. Furthermore, a significant relationship exists between the percentage reduction in Ki and the change in liver disease size at the end of cycle 2 as measured by the change in the sum of all measurable liver lesions (bi-dimensional product). Patients with non-progressive disease have a significantly greater reduction in mean ki. A 50 - 60% reduction in Ki is associated with non-progressive disease.

In general, for the treatment of renal carcinoma, the 4-pyridylmethyl-phthalazine derivative can be given on a continuous basis, for example daily. For 1-(4-chloroanilino)-4-(4-pyridylmethyl)phthalazine, a daily oral dose in the range from 300 mg to 4000 mg, for example in the range from 300 mg/day to 2000 mg/day or 300 mg/day to 1000 mg/day, in particular 300, 500, 750, 1000, 1500 or 2000 mg/day, are contemplated as a pharmaceutically effective dose. However, other administration schedules are also likely to be effective and are included within the scope of the present invention. When 1-(4-chloroanilino)-4-(4-pyridylmethyl)phthalazine is administered as a pharmaceutically acceptable salt, an equivalent amount of the free base (i.e. one equivalent to the amounts described above) is administered.

In a COMBINATION OF THE INVENTION, the 4-pyridylmethyl-phthalazine derivative, in particular 1-(4-chloroanilino)-4-(4-pyridylmethyl)phthalazine or a pharmaceutically acceptable salt thereof, can be given on a continuous basis, for example daily, during and subsequent to the chemotherapy. A daily oral dose in the range from 500 mg to 4000 mg, for example in the range from 500 mg/day to 2000 mg/day, in particular 1000, 1500 or 2000 mg/day, are contemplated. However, other administration schedules are also included within the scope of the present invention. When the 4-pyridylmethyl-phthalazine derivative, in particular 1-(4-chloroanilino)-4-(4-pyridylmethyl)phthalazine, is administered as a salt form, the above daily oral dosage ranges are adjusted so that an equivalent amount of free base administered.

When the combination partners employed in the COMBINATION OF THE INVENTION are applied in the form as marketed as single drugs, their dosage and mode of administration can take place in accordance with the information provided on the package insert of the respective marketed drug in order to result in the beneficial effect described herein, if not mentioned herein otherwise.

The chemotherapy is generally administered according to established administration regimen. Such administration regimens, for example the deGramont regimen for colorectal cancer, are known in the art. In a specific embodiment, the chemotherapy comprises the administration of oxaliplatin, folinic acid and 5-fluorouracil according to an established administration regimen, such as those known in the art. A particular chemotherapy regimen whereby 85 mg/m² of oxaplatin is administered on day 1, 200 mg/m² of folinic acid is given as a 2 hour infusion on days 1 and 2, and 5-fluorouracil is administered as a bolus at a dose of 400 mg/m² followed by 600 mg/m² over 22 hours on days 1 and 2 and is given every 14 days is particularly useful.

5-Fluorouracil may be administered to a human in a dosage range varying from 50 to 1000 mg/m²day, e.g. 500 mg/m²day.

Capecitabine may be administered to a human in a dosage range varying from 10 to 1000 mg/m²day.

Gemcitabine hydrochloride may be administered to a human in a dosage range varying from 10 to 1000 mg/week.

Methotrexate may be administered to a human in a dosage range varying from 5 to 500 mg/m²day.

ZD 1694 (RALTITREXED^{™}) can be administered to a human in a dosage range varying from 2.0 to 4.0 mg/m², e.g., 3.5 mg/m², every 3 weeks as a 15 minute infusion.

Carboplatin may be administered intravenously to a human in a dosage range varying from 100 to 400, e.g. 200, mg/m² body surface about every four to six weeks.

Oxaliplatin may be administered intravenously to a human in a dosage range varying from 25 to 135, e.g. 45 or 85, mg/m² body surface every two to three weeks.

Cisplatin may be administered to a human in a dosage range varying from 25 to 100 mg/m² about every three weeks.

Topotecan may be administered to a human in a dosage range varying from 1 to 5 mg/m² day.

Irinotecan may be administered to a human in a dosage range varying from 50 to 350 mg/m²day.

Fadrozole may be administered orally to a human in a dosage range varying from 0.5 to 10 mg/day, preferably from 1 to 2.5 mg/day

Exemestane may be administered orally to a human in a dosage range varying from 5 to 200 mg/day, preferably from 10 to 25 mg/day, or parenterally from 50 to 500 mg/day, preferably from 100 to 250 mg/day. If the drug shall be administered in a separate pharmaceutical composition, it can be administered in the form disclosed in GB 2,177,700.

Formestane may be administered parenterally to a human in a dosage range varying from 100 to 500 mg/day, preferably from 250 to 300 mg/day.

Anastrozole may be administered orallly to a human in a dosage range varying from 0.25 to 20 mg/day, preferably from 0.5 to 2.5 mg/day.

Aminogluthemide may be administered to a human in a dosage range varying from 200 to 500 mg/day.

Tamoxifen citrate may be administered to a human in a dosage range varying from 10 to 40 mg/day.

Vinblastine may be administered to a human in a dosage range varying from 1.5 to 10 mg/m²day.

Vincristine sulfate may be administered parenterally to a human in a dosage range varying from 0.025 to 0.05 mg/kg body weight * week.

Vinorelbine may be administered to a human in a dosage range varying from 10 to 50 mg/m² day.

Etoposide phosphate may be administered to a human in a dosage range varying from 25 to 115 mg/m²day, e.g. 56.8 or 113.6 mg/m²day.

Teniposide may be administered to a human in a dosage range varying from 75 to 150 mg about every two weeks.

Doxorubicin may be administered to a human in a dosage range varying from 10 to 100 mg/m²day, e.g. 25 or 50 mg/m²day.

Epirubicin may be administered to a human in a dosage range varying from 10 to 200 mg/m²day.

Idarubicin may be administered to a human in a dosage range varying from 0.5 to 50 mg/m²day.

Mitoxantrone may be administered to a human in a dosage range varying from 2.5 to 25 mg/m²day.

Paclitaxel may be administered to a human in a dosage range varying from 50 to 300 mg/m²day.

Alendronic acid may be administered to a human in a dosage range varying from 5 to 10 mg/day.

Clodronic acid may be administered to a human e.g. in a dosage range varying from 750 to 1500 mg/day.

Etridonic acid may be administered to a human in a dosage range varying from 200 to 400 mg/day.

Ibandronic acid may be administered to a human in a dosage range varying from 1 to 4 mg every three to four weeks.

Risedronic acid may be administered to a human in a dosage range varying from 20 to 30 mg/day.

Pamidronic acid may be administered to a human in a dosage range varying from 15 to 90 mg every three to four weeks.

Tiludronic acid may be administered to a human in a dosage range varying from 200 to 400 mg/day.

Trastuzumab may be administered to a human in a dosage range varying from 1 to 4 mg/m²week.

Bicalutamide may be administered to a human in a dosage range varying from 25 to 50 mg/m²day.

It is one objective of this invention to provide a pharmaceutical composition comprising a quantity, which is jointly therapeutically effective against a proliferative disease comprising the COMBINATION OF THE INVENTION. In this composition, the combination partners (a) and (b) can be administered together, one after the other or separately in one combined unit dosage form or in two separate unit dosage forms. The unit dosage form may also be a fixed combination.

The pharmaceutical compositions for separate administration of the combination partners (a) and (b) and for the administration in a fixed combination, i.e. a single galenical compositions comprising at least two combination partners (a) and (b), according to the invention can be prepared in a manner known per se and are those suitable for enteral, such as oral or rectal, and parenteral administration to mammals (warm-blooded animals), including man, comprising a therapeutically effective amount of at least one pharmacologically active combination partner alone or in combination with one or more pharmaceutically acceptable carries, especially suitable for enteral or parenteral application.

Novel pharmaceutical composition contain, for example, from 10 % to 100 %, preferably from 20 % to 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

A further aspect of the present invention relates to the use of improved regimens for the administration of 1-(4-chloroanilino)-4-(4-pyridylmethyl)phthalazine, or a pharmaceutically acceptable salt thereof, for use in the treatment of patients suffering from solid tumor diseases, including, e.g., renal cancer. According to one inventive regimen 1-(4-chloroanilino)-4-(4-pyridylmethyl)phthalazine, or a pharmaceutically acceptable salt thereof, is administered twice or more daily, for example two or three times daily, in reduced amounts compared with the known once daily administration regimens. Alternatively, the present invention embraces a treatment regimen wherein 1-(4-chloroanilino)-4-(4-pyridylmethyl)phthalazine is administered once daily at a dose in the range from 1000 mg/day to 1400 mg/day, particularly a dose of 1200 mg/day to 1300 mg/day, especially 1250 mg/day. The inventive dosing regimens reduce the toxic effects of 1-(4-chloroanilino)-4-(4-pyridylmethyl)phthalazine and improve the efficacy of the treatment by permitting an effective level of the drug, for example above about 1 micromolar, to be maintained for a longer period.

Thus, the present invention relates to the administration of 1-(4-chloroanilino)-4-(4-pyridylmethyl)phthalazine to a patient, which comprises administering a pharmaceutically effective amount of 1-(4-chloroanilino)-4-(4-pyridylmethyl)phthalazine, or a pharmaceutically acceptable salt thereof, to the patient on a twice daily schedule.

Alternatively, the present invention relates to the administration of 1-(4-chloroanilino)-4-(4-pyridylmethyl)phthalazine to a patient, which comprises administering 1-(4-chloroanilino)-4-(4-pyridylmethyl)phthalazine, or a pharmaceutically acceptable salt thereof, to the patient on a once daily schedule at a dose in the range from 1000 mg/day to 1400 mg/day, particularly a dose of 1200 mg/day to 1300 mg/day, such as 1250 mg/day.

The invention further relates to the use of the claimed combination in the manufacture of a medicament for treating a solid tumor disease in a patient, which comprises administering a pharmaceutically effective amount of 1-(4-chloroanilino)-4-(4-pyridylmethyl)phthalazine, or a pharmaceutically acceptable salt thereof, to the patient on a twice daily schedule, or alternatively on a once daily schedule.

1-(4-Chloroanilino)-4-(4-pyridylmethyl)phthalazine is especially useful for inhibiting metastatic growth of a cancer. Thus, the present invention further relates to its use in the manufactured of a medicament for inhibiting metastatic growth in a patient with a cancer, particularly a solid tumor cancer, which comprises administering a pharmaceutically effective amount of 1-(4-chloroanilino)-4-(4-pyridylmethyl)phthalazine or a pharmaceutically acceptable salt thereof, to the patient on a twice daily schedule. Alternatively, the present invention further relates to its use in the manufactured of a medicament for inhibiting metastatic growth in a patient with a cancer, particularly a solid tumor cancer, which comprises administering a pharmaceutically effective amount of 1-(4-chloroanilino)-4-(4-pyridylmethyl)phthalazine or a pharmaceutically acceptable salt thereof, to the patient on a once daily schedule at a dose in the range from 1000 mg/day to 1400 mg/day, particularly a dose of 1200 mg/day to 1300 mg/day, such as 1250 mg/day.

According to one aspect of the present invention, 1-(4-chloroanilino)-4-(4-pyridylmethyl)-phthalazine is given twice daily on a continuous basis, alone, or during and subsequent to other therapies, for example chemotherapy. A daily oral administration of an amount in the range from 300 mg to 4000 mg, for example in the range from 300 mg/day to 2000 mg/day or 300 mg/day to 1000 mg/day, in particular 300, 500, 750, 1000, 1500 or 2000 mg/day, split into two doses, is contemplated as a pharmaceutically effective amount in the twice daily regimen. A 1000 mg/day dose is given as two 500 mg doses 6 to 12 hours apart, for example about 8 hours apart, and a 2000 mg/day dose is administered as two 1000 mg doses 6 to 8 hours apart, for example 12 hours apart.

In the alternative once daily dosage regimen, a dose in the range from 1000 mg/day to 1400 mg/day, particularly a dose of 1200 mg/day to 1300 mg/day, such as 1250 mg/day is administered once per 24 hour period.

When 1-(4-chloroanilino)-4-(4-pyridylmethyl)phthalazine is administered as a pharmaceutically acceptable salt, an equivalent amount of the free base (i.e. one equivalent to the amounts described above) is administered. In this application, reference to 1-(4-chloroanilino)-4-(4-pyridylmethyl)phthalazine is intended to include pharmaceutically acceptable salts thereof.

The different aspects of the present invention can be combined freely with each other. For example, a renal cancer patient or a patient having another tumor type can be teated by administering 1-(4-chloroanilino)-4-(4-pyridylmethyl)phthalazine on a daily basis or twice daily. The improved regimens of administering 1-(4-chloroanilino)-4-(4-pyridylmethyl)-phthalazine can be applied in monotheraoy or in combination therapy, with other words, 1-(4-chloroanilino)-4-(4-pyridylmethyl)phthalazine is administered alone, or in combination with other therapeutic agents. As a combination therapy, it is administered on a once or twice daily basis as described herein and any other therapeutic agent or agents are administered according to its established administration regimen.

### Example 1

15 Patients with histologically confirmed metastatic colorectal cancer seven of which had previously received adjuvant chemotherapy are treated with a combination of 1-(4-chloroanilino)-4-(4-pyridylmethyl)phthalazine and a chemotherapy regimen. The chemotherapy regimen is 85 mg/m² of oxaplatin on day 1, 200 mg/m² of folinic acid given as a 2 hour infusion on days 1 and 2, and 5-fluorouracil administered as a bolus at a dose of 400 mg/m² followed by 600 mg/m² over 22 hours on days 1 and 2. The chemotherapy regimen is given every 14 days. 1-(4-Chloroanilino)-4-(4-pyridylmethyl)phthalazine is administered continuously as a single daily dose of 500 mg/day, 1000 mg/day or 2000 mg/day. The treatment is well-tolerated. The pharmacokinetics of oxaplatin are not altered by coadministration of 1-(4-chloroanilino)-4-(4-pyridylmethyl)phthalazine. Stable diseases are observed in 2 patients, minor responses in 3 patients and partial responses in 8 patients.

### Example 2

Ten patients having metastatic renal cell carcinoma, six of which had previous biologic therapy, are treated with 300, 750 or 1000 mg/day of 1-(4-chloroanilino)-4-(4-pyridylmethyl)phthalazine administered once a day orally. Seven of the ten patients maintain stable disease for a median of 3 months.

### Example 3

Patients with histologically confirmed advanced solid tumors and measurable disease are orally administered 1-(4-chloroanilino)-4-(4-pyridylmethyl)phthalazine twice daily until disease progression or unacceptable toxicity. Sequential cohorts of patients (3 evaluable at each dose) are treated at total daily doses of 300 mg, 500 mg, 1000 mg and 2000 mg, which was split into two separate doses administered 8 hours apart. The patients are monitored by dynamic contrast enhanced magnetic resonance imaging (DCE-MRI) of tumors to provide a measure of the bi-dimensional transfer constant (Ki) prior to treatment, on day 2 and after every 28 day cycle. In addition, tumor volume is measured by magnetic resonance imaging every 28 days, and full pharmacokinetic profiles are obtained on days 1 and 28. Treatment is well tolerated with no drug-related SAEs. The following toxicities are found among the patients administered the 300 mg, 500 mg and 1000 mg daily doses that are entered: transient grade 3 elevations of liver transaminases (2 patients), grade 1 lethargy (one patient), grade 2 lightheadedness (4 patients). DCE-MRI reveals the following reductions compared with baseline (median % reduction days 2.28 respectively 300 mg - 68.3, 500 mg - 72.98). The pharmacokinetic study shows a mean Cmin (ng/ml) and area under the curve (AUC)(h.ng.ml) respectively of 300 mg - 7.7, 82; 500 mg - 4.3, 46; 1000 mg - 27, 370. The data from the study indicates a biological effect in reducing tumor perfusion/vascular permeability and slowing tumor growth.

### Example 4

Patients with histologically confirmed advanced solid tumors and measurable disease are orally administered 1-(4-chloroanilino)-4-(4-pyridylmethyl)phthalazine once daily until disease progression or unacceptable toxicity. Sequential cohorts of patients (3 evaluable at each dose) are treated at total daily doses of 50 mg, 150mg, 300 mg, 500 mg, 750 mg, 1000 mg, 1200 mg, 1500 mg and 2000 mg administered as a single daily dose. Full Pharmacokinetic (PK) sampling is performed at predose, and days 1, 15 and 28. DCE-MRI, which reflects the change in tumor perfusion and vascular permeability, are performed at baseline, and days 2 and 28. For all evaluable MRI scans, contrast enhancement for the whole tumour are assessed by calculating the bi-directional transfer constant (Ki). Plasma VEGF, a proangiogenic factor produced by the tumor cells, reflects the hypoxic status of the tumor and is determined at baseline, and on days 1, 8, 15, 22 and 28. Of a total of 76 patients from two Phase I studies, 22 patients with colorectal cancer and liver metastasis were evaluable for DCE-MRI analysis and 63 patients with advanced cancers for plasma VEGF and PK analysis. Using SWOG response criteria, non-progressive disease was defined as ≥ 2 months stable disease.

1-(4-chloroanilino)-4-(4-pyridylmethyl)phthalazine is rapidly absorbed (Tₘₐₓ 1 to 2.5 hours). At steady-state, which was achieved by day 15, the systemic exposure (AUC) is approximately 30% decreased compared to day 1. Dose proportional increase in exposure is observed up to 1000 mg/day. The terminal half-life is 3 to 6 hours. No dose-limiting toxicity is observed up to 2000 mg/day.

A relationship exists between the percentage of baseline Ki at days 2 and 28 and the increase in dose (day 2: p=0.01; day 28: p=0.0003), exposure (AUC; day 2: p<0.0001; day 28: p=0.003), and plasma concentration (Cₘᵢₙ; day 2: p=0.0003; day 28: p<0.0001) as determined by the Spearman Rank Correlation. Furthermore, a relationship exists between the percent reduction in Ki at days 2 and 28 and the change in size of liver metastases (day 2: p=0.004; day 28: p=0.0001) at the end of day 56 as measured by the change in the sum of all measurable liver lesions (bi-dimensional product). Patients with non-progressive disease have a significantly greater reduction in mean Ki (day 2: p=0.004; day 28: p=0.006). A 50 - 60% reduction in Ki is associated with non-progressive disease. Exposure is approximately 114 hr·µM based on exposure-response modeling. Accounting for a PK variability, a dose with the lower limit (95% Cl) of exposure at 114 hr·µM should be the optimal dose, and thus a daily dose of 1250 mg is recommended as the biologically active dose. Supporting the selected biologically active dose is the dose-dependent acute rise in plasma VEGF level in patients with non-progressive disease. Responders who received doses of >1000 mg/day achieved up to 5 fold rise in plasma VEGF levels within the first 28 days of treatment.

## Claims

1. A combination which comprises (a) a 4-pyridyimethyl-phthalazine antiangiogenic agent and (b) a platinum compound and an antineoplastic antimetabolite and, optionally. folinic acid, wherein the active ingredients are present in each case in free form or in the form of a pharmaceutically acceptable salt, and optionally at least one pharmaceutically acceptable carrier, for simultaneous, separate or sequential use.

2. Combination according to daim 1 comprising (a) a 4-pyridyimethyl-phthalazine antiangiogenic agent and (b) a platinum compound, 5-fluorouracil and folinic acid.

3. Combination according to claim 1 comprising (a) a 4-pyridylmethyl-phthalazine antiangiogenic agent and (b) a platinum compound, capecitabine and folinic acid.

4. A pharmaceutical composition comprising a combination according to any one of claims 1 to 3 and at least one pharmaceutically acceptable carrier.

5. A combination as defined in any one of claims 1 to 3 for use in the treatment of a proliferative disease.

6. Use of a combination as defined in any one of claims 1 to 3 for the preparation of a medicament for the treatment of a proliferative disease.

7. The use according to claim 6 wherein the 4-pyridylmethyl-phthalazine antiangiogenic agent is 1-(4-chloroanilino)-4-(4-pyridylmethyl)phthalazine or a pharmaceutically acceptable salt thereof.

8. The use according to claim 7 wherein a dose in the range from 500 mg/day to 4000 mg/day of 1-(4-chloroanilino)-4-(4-pyridylmethyl)phthalazine, or an equivalent amount of a salt thereof, is administered daily.

9. The use according to claim 8 wherein the range is from 500 mg/day to 2000 mg/day.

10. The use according to daim 8 wherein 500, 1000, 1500 or 2000 mg/day of 1-(4-chloroanilino)-4-(4-pyridylmethyl)phthalazine, or an equivalent amount of a salt thereof, are administered.

11. The use according to any one of claims 7 to 10 wherein the proliferative disease is a solid tumor disease.

12. The use according to claim11 wherein the solid tumor disease is colorectal cancer.

## Patentansprüche

1. Kombination, umfassend (a) ein antiangiogen wirksames 4-Pyridylmethylphthalazin und (b) eine Platinverbindung und einen antineoplastischen Antimetabolit und optional Folinsäure, worin die Wirkstoffe jeweils in freier Form oder in Form eines pharmazeutisch annehmbaren Salzes vorhanden sind, und optional wenigstens einen pharmazeutisch annehmbaren Träger, zur simultanen, separaten oder sequenziellen Anwendung.

2. Kombination nach Anspruch 1, umfassend (a) ein antiangiogen wirksames 4-Pyridylmethylphthalazin und (b) eine Platinverbindung, 5-Fluorouracil und Folinsäure.

3. Kombination nach Anspruch 1, umfassend (a) ein antiangiogen wirksames 4-Pyridylmethylphthalazin und (b) eine Platinverbindung, Capecitabin und Folinsäure.

4. Pharmazeutische Zusammensetzung, umfassend eine Kombination nach einem der Ansprüche 1 bis 3 und wenigstens einen pharmazeutisch annehmbaren Träger.

5. Kombination gemäß der Definition nach einem der Ansprüche 1 bis 3 zur Verwendung für die Behandlung einer proliferativen Krankheit.

6. Verwendung einer Kombination gemäß der Definition nach einem der Ansprüche 1 bis 3 zur Herstellung eines Arzneimittels für die Behandlung einer proliferativen Krankheit.

7. Verwendung nach Anspruch 6, worin das antiangiogen wirksame 4-Pyridylmethylphthalazin 1-(4-Chloranilino)-4-(4-pyridylmethyl)phthalazin oder ein pharmazeutisch annehmbares Salz hiervon ist.

8. Verwendung nach Anspruch 7, worin eine Tagesdosis im Bereich von 500 mg/Tag bis 4000 mg/Tag 1-(4-Chloranilino)-4-(4-pyridylmethyl)phthalazin oder eine äquivalente Menge eines Salzes hiervon verabreicht wird.

9. Verwendung nach Anspruch 8, worin die Tagesdosis im Bereich von 500 mg/Tag bis 2000 mg/Tag liegt.

10. Verwendung nach Anspruch 8, worin 500, 1000, 1500 oder 2000 mg/Tag 1-(4-Chloranilino)-4-(4-pyridylmethyl)phthalazin oder eine äquivalente Menge eines Salzes hiervon verabreicht werden.

11. Verwendung nach einem der Ansprüche 7 bis 10, worin die proliferative Krankheit eine solide Tumorkrankheit ist.

12. Verwendung nach Anspruch 11, worin die Krankheit durch einen soliden Tumor ein Colorektalkrebs ist.

## Revendications

1. Combinaison comprenant (a) un agent antiangiogénique 4-pyridylméthyl-phtalazine et (b) un composé platine et un anti-métabolite anti-néoplasique et, éventuellement, l'acide folinique, **caractérisée en ce que** les ingrédients actifs sont présents, dans chacun des cas, sous forme libre ou sous forme d'un sel pharmaceutiquement acceptable et, éventuellement au moins un véhicule pharmaceutiquement acceptable ; destinée à une utilisation simultanée, séparée ou séquentielle.

2. Combinaison selon la revendication 1 comprenant (a) un agent antiangiogénique 4-pyridylméthyl-phtalazine et (b) un composé platine, du 5-fluorouracile et de l'acide folinique.

3. Combinaison selon la revendication 1 comprenant (a) un agent antiangiogénique 4-pyridylméthyl-phtalazine et (b) un composé platine, de la capécitabine et de l'acide folinique.

4. Composition pharmaceutique comprenant une combinaison selon l'une des revendications 1 à 3 et au moins un véhicule pharmaceutiquement acceptable.

5. Combinaison telle que définie à l'une quelconque des revendications 1 à 3 destinée à être employée pour le traitement d'une maladie proliférative.

6. Utilisation d'une combinaison telle que définie à l'une quelconque des revendications 1 à 3 pour la préparation d'un médicament pour le traitement d'une maladie proliférative.

7. Utilisation selon la revendication 6, **caractérisée en ce que** l'agent antiangiogénique 4-pyridylméthyl-phtalazine est 1-(4-chloroanilino)-4-(4-pyridylméthyl)phtalazine ou un de ses sels pharmaceutiquement acceptables.

8. Utilisation selon la revendication 7, **caractérisée en ce qu'**un dosage compris dans la gamme allant de 500 mg/jour à 4 000 mg/jour de 1-(4-chloroanilino)-4-(4-pyridylméthyl)phtalazine ou, d'une quantité équivalente d'un de ses sels, est administré quotidiennement.

9. Utilisation selon la revendication 8, **caractérisée en ce que** le dosage compris dans la gamme allant de 500 mg/jour à 2 000 mg/jour.

10. Utilisation selon la revendication 8, **caractérisée en ce que** 500, 1 000, 1 500 ou 2 000 mg/jour de 1-(4-chloroanilino)-4-(4-pyridylméthyl) phtalazine ou, d'une quantité équivalente d'un de ses sels, sont administrés.

11. Utilisation selon l'une quelconque des revendications 7 à 10, **caractérisée en ce que** la maladie proliférative est une maladie tumorale solide.

12. Utilisation selon la revendication 11, **caractérisée en ce que** la maladie tumorale solide est un cancer du colon et du rectum.
